# EUROPÄISCHE PATENTSCHRIFT

(11) **EP 3 636 203 B1**
(45) Veröffentlichungstag und Bekanntmachung des Hinweises auf die Patenterteilung: **04.05.2022**
(21) Anmeldenummer: 19210529.4
(22) Anmeldetag: 27.03.2015
(51) Int. Cl.: A61C 1/08, A61C 1/00, A61B 17/20

(54) **MEDIZINISCHES ODER DENTALES INSTRUMENTENTEIL, BEHANDLUNGSVORRICHTUNG UND VERFAHREN ZUM BETRIEB**
MEDICAL OR DENTAL INSTRUMENT PORTION, TREATMENT DEVICE AND METHOD OF OPERATION
PARTIE D'INSTRUMENT MÉDICAL OU DENTAIRE, DISPOSITIF DE TRAITEMENT ET MÉTHODE DE FONCTIONNEMENT

(43) Veröffentlichungstag der Anmeldung: 15.04.2020
(62) Teilanmeldung aus: 15161217.3
(73) Patentinhaber: W & H Dentalwerk Bürmoos GmbH, 5111 Bürmoos (AT)
(72) Erfinder: PRUCKNER, Christian, 1190 Wien (AT); SILBERER, Bernhard, 5152 Michaelbeuern (AT); EIBL, Johann, 5230 Mattighofen (AT)
(74) Vertreter: Benda, Ralf

(56) Entgegenhaltungen:
- EP-A1- 2 184 028
- EP-A1- 2 514 386
- EP-A1- 2 727 552
- US-A1- 2003 165 794

## Beschreibung

Die vorliegende Erfindung betrifft ein medizinisches oder dentales Instrumententeil mit einer mit elektrischer Energie betreibbaren Speichervorrichtung zur Speicherung von Identifikationsdaten und / oder Betriebsdaten und / oder Pflegedaten des Instrumententeils.

Ein medizinisches oder dentales Instrumententeil mit einer mit elektrischer Energie betreibbaren Speichervorrichtung ist zum Beispiel aus der Patentanmeldung US 2003/0165794 A1 bekannt. Die Speichervorrichtung ist Teil einer Identifikationssignal-Ausgabevorrichtung zum aktiven Ausgeben von Identifikationssignalen, um das Instrumententeil zu identifizieren, so dass ein Schaltkreis einer Steuer-, Regel- oder Versorgungseinheit automatisch das Instrumententeil erkennt und entsprechend versorgt oder antreibt. Die Energieversorgung und Kommunikation zwischen der Speichervorrichtung und dem Antriebsschaltkreis erfolgt drahtgebunden über elektrische Leitungen, welche die Speichervorrichtung und die Steuer-, Regel- oder Versorgungseinheit elektrisch verbinden. Eine dieser elektrischen Leitungen ist als gemeinsame elektrische Leitung bezeichnet. Des Weiteren ist an einem als Motorhandstück ausgeführten Instrumententeil ein elektrisches Verbindungsterminal für eine Lampe vorgesehen.

Der vorliegenden Erfindung liegt die Aufgabe zugrunde den Aufbau eines medizinischen oder dentalen Instrumententeils in Hinblick auf die drahtgebundene Energieversorgung und Kommunikationsverbindung/Datenübertragung zwischen der Speichervorrichtung und der Steuer-, Regel- oder Versorgungseinheit zu verbessern. Insbesondere soll der Aufbau des Instrumententeils einfach sein und die zur Energieversorgung und Kommunikationsverbindung zwischen der Speichervorrichtung und der Steuer-, Regel- oder Versorgungseinheit benötigten Bauteile wenig Platz einnehmen und eine zuverlässige Energieversorgung und Datenübertragung gewährleisten.

Diese Aufgabe wird gemäß der vorliegenden Erfindung durch ein medizinisches oder dentales Instrumententeil mit den Merkmalen des Anspruchs 1, durch eine medizinische oder dentale Behandlungsvorrichtung mit den Merkmalen des Anspruchs 12 und durch ein Verfahren zum Betrieb eines medizinischen oder dentalen Instrumententeils oder einer medizinischen oder dentalen Behandlungsvorrichtung mit den Merkmalen des Anspruchs 15 gelöst. Besonders vorteilhafte Ausführungsformen sind in den Unteransprüchen angeführt.

Das medizinische oder dentale Instrumententeil umfasst: eine Kupplungsvorrichtung zur Verbindung des Instrumententeils mit einer Steuer-, Regel- oder Versorgungseinheit, eine mit elektrischer Energie betreibbare Speichervorrichtung zur Speicherung von Identifikationsdaten und / oder Betriebsdaten und / oder Pflegedaten des Instrumententeils und zwei elektrische Leitungen, die für die Versorgung einer an dem Instrumententeil vorgesehenen oder mit dem Instrumententeil verbundenen oder verbindbaren Beleuchtungsvorrichtung mit elektrischer Energie zum Betrieb der Beleuchtungsvorrichtung und für die Versorgung der Speichervorrichtung mit elektrischer Energie zum Betrieb der Speichervorrichtung vorgesehen sind.

Durch das Vorsehen der zwei elektrischen Leitungen, die für die Versorgung der Beleuchtungsvorrichtung mit elektrischer Energie zum Betrieb der Beleuchtungsvorrichtung und für die Versorgung der Speichervorrichtung mit elektrischer Energie zum Betrieb der Speichervorrichtung vorgesehen sind, wird der Aufbau des Instrumententeils erheblich vereinfacht. So wird zum Beispiel die Anzahl an elektrischen Leitungen in dem Instrumententeil reduziert. Insbesondere wird der Aufbau der Kupplungsvorrichtung zur Verbindung des Instrumententeils mit einer Steuer-, Regel- oder Versorgungseinheit außerordentlich vereinfacht, da die Anzahl an elektrischen Kontakten an der Schnittfläche der Kupplungsvorrichtung reduziert ist.

Die zwei elektrischen Leitungen, die für die Versorgung der an dem Instrumententeil vorgesehenen oder mit dem Instrumententeil verbindbaren Beleuchtungsvorrichtung mit elektrischer Energie zum Betrieb der Beleuchtungsvorrichtung und für die Versorgung der Speichervorrichtung mit elektrischer Energie zum Betrieb der Speichervorrichtung vorgesehen sind, sind vorzugsweise als gemeinsame elektrische Leitungen ausgebildet, insbesondere als gemeinsame elektrische Leitungen für die Beleuchtungsvorrichtung und die Speichervorrichtung. Die Beleuchtungsvorrichtung und die Speichervorrichtung sind somit insbesondere elektrisch mit den (gemeinsamen) elektrischen Leitungen verbunden. Besonders bevorzugt ist eine Verzweigung vorgesehen, von welcher sich eine erste elektrische Zweigleitung zu der Speichervorrichtung und eine zweite elektrische Zweigleitung zu der Beleuchtungsvorrichtung erstreckt.

Die zwei (gemeinsamen) elektrischen Leitungen sind vorzugsweise als Drahtleitung und / oder als auf einer Leiterplatte gedruckte elektrische Leitung ausgebildet.

Unter dem Begriff ,medizinisches oder dentales Instrumententeil' wird im Folgenden insbesondere verstanden: ein mit einer Hand haltbares medizinisches oder dentales Element, ein Handgriffelement, ein gerades Handstück, ein gewinkeltes oder gebogenes Handstück oder Winkelstück, ein Adapter, ein Kupplungselement, ein Antriebselement, insbesondere ein Luftmotor oder ein E-Motor, oder ein Versorgungsschlauch oder ein Teil der im Vorstehenden genannten Elemente.

Vorzugsweise umfasst das Instrumententeil ein medizinisches oder dentales Handstück oder zumindest einen Teil davon, das aufweist: ein in Drehung versetzbares Antriebselement zum Antrieb eines mit dem Handstück lösbar verbindbaren Werkzeugs, eine Kupplungsvorrichtung zur Verbindung des Handstücks mit einer Steuer-, Regel- oder Versorgungseinheit, eine mit elektrischer Energie betreibbare Speichervorrichtung zur Speicherung von Identifikationsdaten und / oder Betriebsdaten und / oder Pflegedaten des Handstücks, eine mit elektrischer Energie betreibbare Beleuchtungsvorrichtung zur Abgabe von Strahlung auf eine Behandlungsstelle und zwei (gemeinsame) elektrische Leitungen, die für die Versorgung der Beleuchtungsvorrichtung mit elektrischer Energie zum Betrieb der Beleuchtungsvorrichtung und der Speichervorrichtung mit elektrischer Energie zum Betrieb der Speichervorrichtung vorgesehen sind.

Die Kupplungsvorrichtung zur Verbindung des Instrumententeils mit einer Steuer-, Regel- oder Versorgungseinheit ist insbesondere als lösbare Kupplungsvorrichtung ausgebildet, so dass das Instrumententeil von der Steuer-, Regel- oder Versorgungseinheit trennbar ist. Die Kupplungsvorrichtung ist zum Beispiel als Steckkupplung oder als Drehkupplung ausgebildet, so dass das Instrumententeil relativ zu der Steuer-, Regel- oder Versorgungseinheit drehbar ist, wenn das Instrumententeil mit dem Steuer-, Regel- oder Versorgungseinheit über die Kupplungsvorrichtung verbunden ist.

Die Kupplungsvorrichtung umfasst insbesondere eine Stirnfläche, an welcher vorzugsweise zwei elektrische Kontakte angeordnet sind, wobei jeweils ein elektrischer Kontakt mit einer der zwei gemeinsamen elektrischen Leitungen verbunden ist. Vorzugsweise ist in entsprechender Weise ein elektrischer Kontakt vorgesehen, der mit einer elektrischen Leitung für die Datenübertragung verbunden ist.

Vorzugsweise sind die elektrischen Kontakte lösbar mit der Steuer-, Regel- oder Versorgungseinheit verbunden. Die elektrischen Kontakte sind zum Beispiel als stiftförmiger elektrischer Kontakt, als Federkontakt, als Schleifkontakt oder als ringförmiger, ein Bauteil der Kupplungsvorrichtung umgebender elektrischer Kontakt ausgebildet.

Die Speichervorrichtung umfasst entweder einen nur lesbaren Speicher (ROM) oder vorzugsweise einen beschreibbaren und lesbaren Speicher. Die Speichervorrichtung ist insbesondere als digitale Speichervorrichtung zur Speicherung und / oder zum Auslesen und / oder zum Verarbeiten digitaler Daten ausgebildet.

Vorzugsweise sind in der Speichervorrichtung des Instrumententeils insbesondere Identifikationsdaten und / oder Betriebsdaten und / oder Pflegedaten gespeichert oder speicherbar, die jenem Instrumententeil zugeordnet sind, in dem die Speichervorrichtung angeordnet ist. Alternativ ist es auch denkbar, dass in der Speichervorrichtung ausschließlich oder zusätzlich Identifikationsdaten und / oder Betriebsdaten und / oder Pflegedaten von einem anderen als jenem Instrumententeil, in dem die Speichervorrichtung angeordnet ist, speicherbar oder gespeichert sind.

Die Speichervorrichtung umfasst vorzugsweise ein Speicherelement und einen Mikrocontroller oder Mikrocomputer, die wahlweise als ein Bauteil oder als zwei getrennte, elektrisch miteinander verbundene Bauteile ausgebildet sind. Wenn das Speicherelement und der Mikrocontroller getrennt voneinander ausgebildet sind, ist es möglich sie an unterschiedlichen Stellen des Instrumententeils zu positionieren, wodurch die Anordnung der beiden Bauteile in einem Instrumententeil mit geringem inneren Raumvolumen erleichtert wird.

Die Speichervorrichtung mit dem Mikrocontroller ist vorzugsweise zum aktiven Ausgeben eines digitalen Signals oder von digitalen Daten ausgebildet. Die Speichervorrichtung mit dem Mikrocontroller umfasst insbesondere auch Software, die es ermöglicht Daten zu empfangen, abzusenden, zu verarbeiten, zu speichern und / oder eigenständige Steuerschritte durchzuführen.

Die Versorgung der Beleuchtungsvorrichtung und der Speichervorrichtung mit elektrischer Energie sowie vorzugsweise die (digitale) Datenübertragung über die (gemeinsamen) elektrischen Leitungen ist/sind zum Beispiel durch Aufmodulieren und / oder durch digitales Multiplexen und / oder durch zeitlich versetztes Senden oder Übertragen von digitalen Daten und / oder elektrischer Energie realisierbar, insbesondere mit Hilfe des Mikrocontrollers der Speichervorrichtung.

Die an dem Instrumententeil vorgesehene oder mit dem Instrumententeil verbindbare Beleuchtungsvorrichtung umfasst vorzugsweise eine oder mehrere Lichtquellen, zum Beispiel optische Halbleiterelemente, insbesondere Leuchtdioden (LEDs). Vorzugsweise umfasst die Beleuchtungsvorrichtung mehrere Lichtquellen, wobei zumindest eine erste Lichtquelle (LED) eine erste Wellenlänge oder einen ersten Wellenlängenbereich und zumindest eine zweite Lichtquelle (LED) eine zweite Wellenlänge oder einen zweiten Wellenlängenbereich emittiert, die von der ersten Wellenlänge oder dem ersten Wellenlängenbereich unterschiedlich ist. Bevorzugt sind die erste Lichtquelle und die zweite Lichtquelle in unterschiedlichen elektrischen Stromrichtungen angeordnet. Besonders bevorzugt ist an dem Instrumententeil, insbesondere mit dem Mikrocontroller verbunden oder als Teil des Mikrocontrollers, oder an einem mit dem Instrumententeil verbundenen oder verbindbaren Element, zum Beispiel der Steuer-, Regel- oder Versorgungseinheit, eine Schaltvorrichtung zum wahlweisen Betrieb der ersten Lichtquelle oder der zweiten Lichtquelle und / oder zur wahlweisen Emission von elektromagnetischer Strahlung durch die erste Lichtquelle oder durch die zweite Lichtquelle vorgesehen. Besonders bevorzugt steuert die Schaltvorrichtung den wahlweisen Betrieb der ersten Lichtquelle oder der zweiten Lichtquelle über die zumindest eine (gemeinsame) elektrische Leitung. Besonders bevorzugt ist die Schaltvorrichtung als elektrische Schaltvorrichtung ausgebildet, zum Beispiel als H-Brücke.

Wenn das Instrumententeil als medizinisches oder dentales Handstück ausgebildet ist, so wie dies im Vorstehenden beschrieben ist, so ist die Beleuchtungsvorrichtung vorzugsweise anschließend an einen oder an einem Kopfteil des Handstücks, in dem die Werkzeughalterung für das lösbar verbindbare Werkzeug gelagert ist, angeordnet. Insbesondere umfasst die Beleuchtungsvorrichtung mehrere optische Halbleiterelemente, die ringförmig um eine Werkzeugaufnahmeöffnung des Kopfteils positioniert sind.

Alternativ umfasst das Instrumententeil mit der Speichervorrichtung, insbesondere mit dem Mikrocontroller, keine Beleuchtungsvorrichtung, sondern nur (gemeinsame) elektrische Leitungen, die neben der Versorgung der Speichervorrichtung mit elektrischer Energie auch zur Versorgung der Beleuchtungsvorrichtung mit elektrischer Energie vorgesehen sind. Die Beleuchtungsvorrichtung ist in einem anderen Element angeordnet, das mit dem Instrumententeil mit der Speichervorrichtung derart verbindbar oder verbunden ist, dass die Beleuchtungsvorrichtung über die (gemeinsamen) elektrischen Leitungen mit elektrischer Energie versorgbar ist.

Erfindungsgemäß sind zwei (gemeinsame) elektrische Leitungen für die Versorgung der Beleuchtungsvorrichtung mit elektrischer Energie zum Betrieb der Beleuchtungsvorrichtung und der Speichervorrichtung mit elektrischer Energie zum Betrieb der Speichervorrichtung vorgesehen. Die Versorgung der Beleuchtungsvorrichtung und der Speichervorrichtung mit elektrischer Energie erfolgen damit ausschließlich über diese zwei (gemeinsamen) elektrischen Leitungen.

Erfindungsgemäß ist eine separate Leitung zum Übertragen von Identifikationsdaten und / oder Betriebsdaten und / oder Pflegedaten zwischen der Speichervorrichtung und der mit dem Instrumententeil verbindbaren Steuer-, Regel- oder Versorgungseinheit vorgesehen. Diese separate Leitung (im Folgenden auch Datenleitung genannt) ist somit, vorzugsweise ausschließlich, für die Datenübertragung zwischen der Speichervorrichtung und der Steuer-, Regel- oder Versorgungseinheit, jedoch nicht für die Versorgung der Beleuchtungsvorrichtung und der Speichervorrichtung mit elektrischer Energie vorgesehen. Die separate Datenleitung ist als elektrische Leitung zum Übertragen elektrischer (Daten-)Signale ausgebildet. Besonders bevorzugt bildet die Datenleitung eine direkte Verbindung zwischen dem Mikrocontroller der Speichervorrichtung des Instrumententeils und der Steuer-, Regel- oder Versorgungseinheit, insbesondere einem weiteren, in der Steuer-, Regel- oder Versorgungseinheit angeordneten Mikrocontroller.

Erfindungsgemäß sind somit drei Leitungen zur Versorgung der Beleuchtungsvorrichtung und der Speichervorrichtung mit elektrischer Energie sowie zur Übertragung der Identifikationsdaten und / oder Betriebsdaten und / oder Pflegedaten zwischen der Speichervorrichtung und der Steuer-, Regel- oder Versorgungseinheit vorgesehen. Diese drei Leitungen umfassen insbesondere zumindest zwei elektrische Leitungen für die Versorgung der Beleuchtungsvorrichtung und der Speichervorrichtung mit elektrischer Energie und eine dritte elektrische Leitung zur Datenübertragung. In entsprechender Weise sind drei elektrische Kontakte an der Kupplungsvorrichtung des Instrumententeils vorgesehen, wobei jeder Kontakt mit einer der drei Leitungen verbunden ist.

Vorzugsweise sind die Beleuchtungsvorrichtung und die Speichervorrichtung mit dem Mikrocontroller, insbesondere einschließlich einer der Speichervorrichtung zugeordneten Spannungsvorrichtung, elektrisch parallel angeordnet.

Vorzugsweise ist ein, insbesondere der Speichervorrichtung zugeordnetes, elektrisches Schaltelement vorgesehen, das ausgebildet ist, Änderungen eines elektrischen Stromparameters, insbesondere der elektrischen Spannung, zur Übertragung von Identifikationsdaten und / oder Betriebsdaten und / oder Pflegedaten zwischen der Speichervorrichtung und der mit dem Instrumententeil verbindbaren Steuer-, Regel- oder Versorgungseinheit zu bewirken. Vorzugsweise ist das elektrische Schaltelement zum Unterstützen des Auslesens oder zum Auslesen von Daten aus der Speichervorrichtung vorgesehen, insbesondere zum Übertragen von Daten von der Speichervorrichtung zu der Steuer-, Regel- oder Versorgungseinheit. Vorzugsweise öffnet und schließt das elektrische Schaltelement einen Schaltkreis zwischen der Speichervorrichtung und der Steuer-, Regel- oder Versorgungseinheit, wodurch ein elektrischer Stromparameter, insbesondere die elektrische Spannung, veränderbar ist, zum Beispiel ein elektrischer Stromparameter der elektrischen Energie zur Versorgung der Beleuchtungsvorrichtung und der Speichervorrichtung. Vorzugsweise ist das elektrische Schaltelement mit den (gemeinsamen) elektrischen Leitungen elektrisch verbunden. Vorzugsweise sind die (gemeinsamen) elektrischen Leitungen Teil dieses Schaltkreises. Wie im Weiteren noch im Detail beschrieben wird, ist die Steuer-, Regel- oder Versorgungseinheit ausgebildet, die Änderung des elektrischen Stromparameters zu empfangen, wobei die Änderung des Stromparameters bzw. die Reaktion der Steuer-, Regel- oder Versorgungseinheit die übertragenen Identifikationsdaten und / oder Betriebsdaten und / oder Pflegedaten, insbesondere digital, definieren.

Vorzugsweise ist das elektrische Schaltelement ausgebildet, zum Auslesen von Daten aus der Speichervorrichtung den im folgenden Absatz beschriebenen Shunt-Widerstand kurzzuschließen.

Vorzugsweise ist ein, insbesondere der Speichervorrichtung zugeordneter, Shunt-Widerstand vorgesehen, der ausgebildet ist, Änderungen der elektrischen Stromstärke zur Übertragung von Identifikationsdaten und / oder Betriebsdaten und / oder Pflegedaten zwischen der Speichervorrichtung und der mit dem Instrumententeil verbindbaren Steuer-, Regel- oder Versorgungseinheit aufzubereiten. Vorzugsweise ist der Shunt-Widerstand zum Abspeichern von Daten in der Speichervorrichtung vorgesehen oder er unterstützt dieses, insbesondere zum Übertragen von Daten von der Steuer-, Regel- oder Versorgungseinheit zu der Speichervorrichtung. Vorzugsweise wandelt der Shunt-Widerstand Werte der elektrischen Stromstärke der Steuer-, Regel- oder Versorgungseinheit, welche Identifikationsdaten und / oder Betriebsdaten und / oder Pflegedaten (digital) definieren, in von der Speichervorrichtung verarbeitbare Spannungswerte, welche Identifikationsdaten und / oder Betriebsdaten und / oder Pflegedaten (digital) definieren. Vorzugsweise ist der Shunt-Widerstand mit den (gemeinsamen) elektrischen Leitungen elektrisch verbunden.

Bevorzugt ist der Shunt-Widerstand alternativ oder zusätzlich zur Stromüberwachung für die Beleuchtungsvorrichtung, insbesondere für das zumindest eine optische Halbleiterelement, ausgebildet. Insbesondere verhindert der Shunt-Widerstand eine Versorgung der Beleuchtungsvorrichtung mit einer zu hohen elektrischen Stromstärke.

Vorzugsweise ist eine der Speichervorrichtung, insbesondere dem Mikrocontroller, zugeordnete Vorrichtung zur Spannungsaufbereitung vorgesehen, die ausgebildet ist, der Speichervorrichtung, insbesondere dem Mikrocontroller, eine konstante elektrische Spannung zuzuführen. Insbesondere bildet die Spannungsaufbereitung eine konstante Spannungsquelle für die Speichervorrichtung, insbesondere den Mikrocontroller. Vorzugsweise ist die Spannungsaufbereitung mit einer Energiequelle, insbesondere mit einer elektrischen Konstantstromquelle, in der Steuer-, Regel- oder Versorgungseinheit elektrisch verbunden, insbesondere über die (gemeinsamen) elektrischen Leitungen. Vorzugsweise versorgt die Energiequelle, insbesondere die Konstantstromquelle, in der Steuer-, Regel- oder Versorgungseinheit die Spannungsaufbereitung mit elektrischer Energie. Vorzugsweise ist die Spannungsaufbereitung derart ausgebildet, dass die Speichervorrichtung, insbesondere der Mikrocontroller, mit einer geringeren elektrischen Spannung versorgt wird als die Beleuchtungsvorrichtung. Vorzugsweise ist die Spannungsaufbereitung in Serie mit der Speichervorrichtung angeordnet.

Vorzugsweise ist eine der Beleuchtungsvorrichtung zugeordnete Vorrichtung zur Spannungsüberwachung vorgesehen, die ausgebildet ist, die der Beleuchtungsvorrichtung zugeführte elektrische Spannung zu überwachen. Die Spannungsüberwachung umfasst insbesondere einen Spannungsteiler, der die Eingangsspannung am Instrumententeil überwacht. Der Spannungsteiler weist zum Beispiel zwei elektrische Widerstände auf, die mit dem Mikrocontroller der Speichervorrichtung verbunden sind.

Gemäß einem Ausführungsbeispiel umfasst das Instrumententeil zumindest einen Sensor, der ausgebildet ist, einen Betriebszustand des Instrumententeils zu detektieren und ein, vorzugsweise elektrisches, Sensorsignal zu erzeugen, wobei der zumindest eine Sensor mit den (gemeinsamen) elektrischen Leitungen elektrisch verbunden ist, so dass das Sensorsignal des Sensors und / oder elektrische Energie zum Betreiben des Sensors über die (gemeinsamen) elektrischen Leitungen übertragbar ist/sind.

Der Sensor weist zum Beispiel einen Temperatursensor zum Messen der Temperatur des Instrumententeils oder eines Abschnitts davon oder zumindest eines in dem Instrumententeil angeordneten Bauteils, insbesondere eines durch ein Antriebselement in Bewegung versetzbaren Bauteils, zum Beispiel eines Lagers, auf. Der Temperatursensor umfasst insbesondere einen elektrisch betreibbaren Temperatursensor, zum Beispiel einen Temperatursensor mit einem Material, dessen elektrischer Widerstand sich mit der Temperatur verändert (z.B. NTC-Temperatursensor), oder einen Infrarot-Temperatursensor.

Der Sensor weist alternativ zum Beispiel einen Drehzahlsensor zum Messen der Drehzahl eines Antriebselements des Instrumententeils oder eines damit verbindbaren Werkzeugs auf. Der Drehzahlsensor ist zum Beispiel als induktiver, kapazitiver oder optischer Drehzahlsensor ausgebildet.

Selbstverständlich kann der Sensor auch ausgebildet sein, andere Parameter zu messen, und zum Beispiel einen Sensor zum Messen einer Leistung, von Kraft oder Druck, eines Drehmoments, einer Lichtintensität oder Wellenlänge, etc. aufweisen.

Vorzugsweise wird das Sensorsignal über die (gemeinsamen) elektrischen Leitungen oder alternativ über die im Vorstehenden beschriebene separate Datenleitung an die Steuer-, Regel- oder Versorgungseinheit, insbesondere an einen darin angeordneten Mikrocontroller, geleitet. Der zumindest eine Sensor ist somit insbesondere derart über die (gemeinsamen) elektrischen Leitungen oder über die separate Datenleitung mit der Steuer-, Regel- oder Versorgungseinheit verbunden, dass die Steuer-, Regel- oder Versorgungseinheit ein Sensorsignal empfängt und verarbeitet. Insbesondere ist die Steuer-, Regel- oder Versorgungseinheit ausgebildet, das Instrumententeil auf Basis eines von dem zumindest einem Sensor erhaltenen Sensorsignal zu steuern, zu regeln oder, zum Beispiel mit einem Betriebsmittel, zu versorgen.

Gemäß einem Ausführungsbeispiel ist eine medizinische oder dentale Behandlungsvorrichtung vorgesehen, die umfasst: ein medizinisches oder dentales Instrumententeil wie im Vorstehenden oder nachfolgend beschrieben, eine elektrische Energiequelle, insbesondere eine Konstantstromquelle, die ausgebildet ist, die Beleuchtungsvorrichtung und die Speichervorrichtung des Instrumententeils mit elektrischer Energie zu versorgen, und eine Steuer-, Regel- oder Versorgungseinheit, die zum Austausch von Identifikationsdaten und / oder Betriebsdaten und / oder Pflegedaten des Instrumententeils mit der Speichervorrichtung des Instrumententeils ausgebildet ist. Vorzugsweise sind das Instrumententeil und die Steuer-, Regel- oder Versorgungseinheit über die Kupplungsvorrichtung lösbar miteinander verbunden.

Vorzugsweise ist in der Steuer-, Regel- oder Versorgungseinheit ein weiterer Mikrocontroller vorgesehen, der mit dem Mikrocontroller des Instrumententeils verbunden ist, wobei die (gemeinsamen) elektrischen Leitungen einen Teil dieser Verbindung bilden. Vorzugsweise ist auch die Energiequelle in der Steuer-, Regel- oder Versorgungseinheit angeordnet und insbesondere über die (gemeinsamen) elektrischen Leitungen mit der Beleuchtungsvorrichtung und der Speichervorrichtung elektrisch verbunden.

Wie im Vorstehenden bereits beschrieben versorgt die Energiequelle, insbesondere die Konstantstromquelle, die Beleuchtungsvorrichtung mit einer konstanten Stromstärke. Die Speichervorrichtung, insbesondere der Mikrocontroller, die eine konstante elektrische Spannung benötigt, wird von der vorzugsweise als Konstantstromquelle ausgebildeten Energiequelle über eine der Speichervorrichtung zugeordnete Vorrichtung zur Spannungsaufbereitung mit elektrischer Energie versorgt. Die Vorrichtung zur Spannungsaufbereitung ist ausgebildet, der Speichervorrichtung eine konstante elektrische Spannung zuzuführen.

Vorzugsweise ist/sind die Steuer-, Regel- oder Versorgungseinheit, insbesondere deren Mikrocontroller, ausgebildet, zum Auslesen der in der Speichervorrichtung gespeicherten Identifikationsdaten und / oder Betriebsdaten und / oder Pflegedaten, insbesondere durch das Schaltelement bewirkte, Änderungen der elektrischen Last oder der elektrischen Spannung zu detektieren oder zu erkennen.

Wie im Vorstehenden bereits beschrieben öffnet und schließt das elektrische Schaltelement vorzugsweise einen Schaltkreis zwischen der Speichervorrichtung und der Steuer-, Regel- oder Versorgungseinheit und schließt insbesondere den der Speichervorrichtung zugeordneten Shunt-Widerstand kurz, wodurch ein elektrischer Stromparameter, insbesondere die elektrische Spannung, welcher die Identifikationsdaten und / oder Betriebsdaten und / oder Pflegedaten (digital) definiert, veränderbar ist. Die Steuer-, Regel- oder Versorgungseinheit, insbesondere deren Mikrocontroller, sind ausgebildet, die von dem Schaltelement bewirkten Änderungen oder Schwankungen zu erkennen oder zu detektieren. Die Änderung des Stromparameters bzw. das Ausgleichen dieser Schwankungen des elektrischen Stromparameters werden von dem Mikrocontroller der Steuer-, Regel- oder Versorgungseinheit erfasst und daraus die von der Speichervorrichtung übertragenen Identifikationsdaten und / oder Betriebsdaten und / oder Pflegedaten erkannt.

Vorzugsweise ist/sind die elektrische Energiequelle, insbesondere die Konstantstromquelle, und / oder die Steuer-, Regel- oder Versorgungseinheit ausgebildet, zum Übertragen von Daten an die Speichervorrichtung die elektrische Stromstärke, welche dem, insbesondere der Speichervorrichtung zugeordneten, Shunt-Widerstand und der Speichervorrichtung zuführbar ist, zu variieren. Der Mikrocontroller der Steuer-, Regel- oder Versorgungseinheit ist insbesondere ausgebildet, die Änderung der elektrischen Stromstärke zu bewirken oder zu steuern. Die Änderung der Stromstärke definiert, insbesondere digital, die Identifikationsdaten und / oder Betriebsdaten und / oder Pflegedaten. Wie im Vorstehenden beschrieben, wandelt vorzugsweise ein Shunt-Widerstand die Werte der elektrischen Stromstärke der Steuer-, Regel- oder Versorgungseinheit, welche Identifikationsdaten und / oder Betriebsdaten und / oder Pflegedaten definieren, in elektrische Spannungswerte um, die von dem Mikrocontroller des Instrumententeils empfangbar und verarbeitbar sind, so dass der Mikrocontroller Daten auf dem Speicherelement der Speichervorrichtung abspeichern kann.

Vorzugsweise ist die Steuer-, Regel- oder Versorgungseinheit ausgebildet, das Instrumententeil auf Basis von in der Speichervorrichtung gespeicherten und aus der Speichervorrichtung ausgelesenen Identifikationsdaten und / oder Betriebsdaten und / oder Pflegedaten und / oder auf Basis des von dem zumindest einem Sensor erhaltenen Sensorsignals zu betreiben. Zum Beispiel ist die Steuer-, Regel- oder Versorgungseinheit ausgebildet, auf Basis dieser Daten einem Instrumententeil die auf dieses Instrumententeil abgestimmte Antriebsenergie und / oder zumindest ein bestimmtes Medium und / oder eine bestimmte Medienmenge zuzuleiten.

Vorzugsweise ist die Steuer-, Regel- oder Versorgungseinheit ausgebildet, auf das Instrumententeil zum Beispiel Daten bezüglich der Dauer des Betriebs des Instrumententeils, der Art seiner Verwendung und / oder der Art und / oder Dauer seiner Reinigung oder Pflege zu übertragen.

Gemäß einem Ausführungsbeispiel ist ein Verfahren zum Betrieb eines medizinischen oder dentalen Instrumententeils oder einer medizinischen oder dentalen Behandlungsvorrichtung vorgesehen, bei dem zwei (gemeinsame) elektrische Leitungen eine an dem Instrumententeil vorgesehene oder mit dem Instrumententeil verbundene oder verbindbare Beleuchtungsvorrichtung zum Betrieb der Beleuchtungsvorrichtung und eine Speichervorrichtung zum Betrieb der Speichervorrichtung mit elektrischer Energie versorgen.

Vorzugsweise versorgen nicht mehr als zwei elektrische Leitungen die Beleuchtungsvorrichtung zum Betrieb der Beleuchtungsvorrichtung und die Speichervorrichtung zum Betrieb der Speichervorrichtung mit elektrischer Energie. Zusätzlich überträgt zumindest eine separate Leitung (Datenleitung) die Identifikationsdaten und / oder Betriebsdaten und / oder Pflegedaten, insbesondere direkt, zwischen der Speichervorrichtung und der mit dem Instrumententeil verbindbaren Steuer-, Regel- oder Versorgungseinheit. Die Datenleitung ist als elektrische Leitung ausgebildet, welche die Daten in Form elektrischer Signale überträgt.

Vorzugsweise übertragen die (gemeinsamen) elektrischen Leitungen zusätzlich ein Sensorsignal des Sensors im Instrumententeil und / oder elektrische Energie zum Betreiben des Sensors, so wie dies im Vorstehenden beschrieben ist.

Vorzugsweise detektiert/detektieren oder erkennt/erkennen die Steuer-, Regel- oder Versorgungseinheit, insbesondere deren Mikrocontroller, zum Auslesen der in der Speichervorrichtung gespeicherten Identifikationsdaten und / oder Betriebsdaten und / oder Pflegedaten durch das Schaltelement bewirkte Änderungen eines elektrischen Stromparameters, insbesondere der elektrischen Spannung. Die Änderung des elektrischen Stromparameters bzw. das Ausgleichen dieser Schwankungen erfasst der Mikrocontroller der Steuer-, Regel- oder Versorgungseinheit und erkennt daraus die von der Speichervorrichtung übertragenen Identifikationsdaten und / oder Betriebsdaten und / oder Pflegedaten, so wie dies im Vorstehenden beschrieben ist.

Vorzugsweise variiert/variieren die elektrische Energiequelle, insbesondere die Konstantstromquelle, und / oder die Steuer-, Regel- oder Versorgungseinheit zum Übertragen von Daten an die Speichervorrichtung die elektrische Stromstärke, welche dem Shunt-Widerstand und der Speichervorrichtung zugeführt wird. Der Shunt-Widerstand wandelt vorzugsweise die Werte der elektrischen Stromstärke der Steuer-, Regel- oder Versorgungseinheit, welche Identifikationsdaten und / oder Betriebsdaten und / oder Pflegedaten definieren, in elektrische Spannungswerte um, welche Identifikationsdaten und / oder Betriebsdaten und / oder Pflegedaten (digital) definieren. Diese elektrischen Spannungswerte werden von dem Mikrocontroller des Instrumententeils empfangen oder verarbeitet und auf das Speicherelement gespeichert, so wie dies im Vorstehenden beschrieben ist.

Vorzugsweise betreibt die Steuer-, Regel- oder Versorgungseinheit das Instrumententeil auf Basis von in der Speichervorrichtung gespeicherten und aus der Speichervorrichtung ausgelesenen Identifikationsdaten und / oder Betriebsdaten und / oder Pflegedaten und / oder auf Basis des von dem zumindest einem Sensor erhaltenen Sensorsignals, so wie dies im Vorstehenden beschrieben ist.

Die Erfindung wird nachfolgend anhand bevorzugter Ausführungsbeispiele und Bezug nehmend auf die beigefügten Zeichnungen erläutert.
Figur 1 zeigt eine medizinische oder dentale Behandlungsvorrichtung, umfassend: ein medizinisches oder dentales Instrumententeil mit einer Beleuchtungsvorrichtung, einer Speichervorrichtung und gemeinsamen elektrischen Leitungen für die Versorgung der Beleuchtungsvorrichtung und der Speichervorrichtung mit elektrischer Energie, eine Antriebsvorrichtung, einen Versorgungsschlauch und eine Steuer-, Regel- oder Versorgungseinheit mit einer elektrischen Energiequelle zur Versorgung der Beleuchtungsvorrichtung und der Speichervorrichtung mit elektrischer Energie.
Figur 2 zeigt das medizinische oder dentale Instrumententeil der Figur 1.
Figur 3 zeigt eine schematische Darstellung eines ersten Ausführungsbeispiels, welches nicht Teil der Erfindung ist, eines elektrischen Schalt- oder Stromkreises mit zumindest einer gemeinsamen elektrischen Leitung für die Versorgung einer Beleuchtungsvorrichtung und einer Speichervorrichtung.
Figur 4 zeigt eine schematische Darstellung eines zweiten Ausführungsbeispiels eines elektrischen Schalt- oder Stromkreises mit gemeinsamen elektrischen Leitungen für die Versorgung einer Beleuchtungsvorrichtung und einer Speichervorrichtung.
Figur 5A-5C zeigen drei unterschiedliche Ausführungsformen einer Beleuchtungsvorrichtung und eines Sensors für einen elektrischen Schalt- oder Stromkreis der Figuren 4 oder 3.

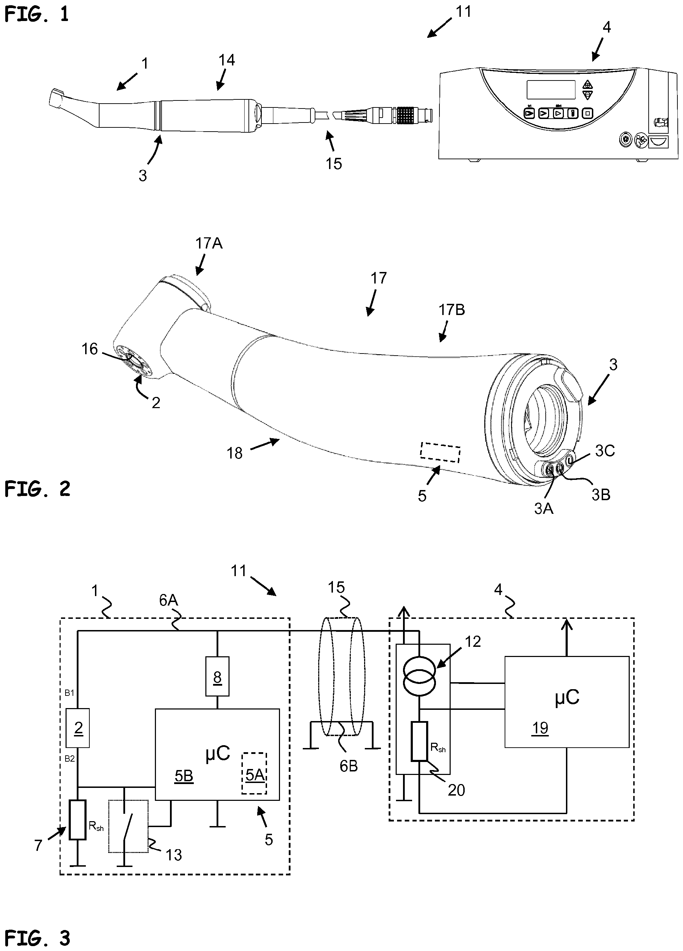

Die Figur 1 zeigt eine medizinische oder dentale Behandlungsvorrichtung 11, die ein medizinisches oder dentales Instrumententeil 1, eine Antriebseinheit 14, eine Steuer-, Regel- oder Versorgungseinheit 4 und einen das Instrumententeil 1 mit der Steuer-, Regel- oder Versorgungseinheit 4 verbindenden Versorgungsschlauch 15 umfasst. Das Instrumententeil 1 ist über eine Kupplungsvorrichtung 3 lösbar mit der Steuer-, Regel- oder Versorgungseinheit 4 verbunden.

Das in der Figur 2 vergrößert dargestellte Instrumententeil 1 ist durch ein gebogenes Handstück oder Winkelstück 17 gebildet. Das Handstück 17 umfasst einen Handstückkopf 17A, in dem eine Werkzeughalterung zur, insbesondere lösbaren, Befestigung eines Werkzeugs angeordnet ist, sowie einen Griffteil 17B zum Halten der Handstücks 17 mit einer Hand. Am freien oder proximalen Ende des Griffteils 17B ist die Kupplungsvorrichtung 3 bzw. zumindest ein Teil davon angeordnet.

Die Antriebseinheit 14 umfasst zum Beispiel einen E-Motor oder einen Luftmotor, die als separates, von dem Handstück 17 lösbares Bauteil (wie in Figur 1 dargestellt) oder in das Handstück 17 integriert ausgebildet sind. Die Antriebseinheit 14 kann jedoch auch ein in das Handstück 17, insbesondere im Handstückkopf 17A, angeordnetes, mit einem Treibgas angetriebenes Laufrad umfassen. Das Handstück 17 kann somit motorbetrieben sein und zumindest eine Antriebswelle zum Übertragen einer Antriebsbewegung auf das Werkzeug aufweisen oder treibgasbetrieben mit einem Laufrad ausgebildet sein. In beiden Fällen umfasst das Handstück 17 zumindest ein in Drehung versetzbares Antriebselement zum Antrieb eines mit dem Handstück verbindbaren Werkzeugs.

Das Handstück 17 umfasst des Weiteren eine Beleuchtungsvorrichtung 2, die vorzugsweise am Handstückkopf 17A oder daran anschließend angeordnet ist. Die in der Figur 2 dargestellte Beleuchtungsvorrichtung 2 umfasst insbesondere mehrere optische Halbleiterelemente (LEDs), die ringförmig um eine Werkzeugaufnahmeöffnung 16 des Handstückkopfs 17A angeordnet sind.

Bevorzugt sind des Weiteren um die Werkzeugaufnahmeöffnung 16, vorzugsweise alternierend mit den optischen Halbleiterelementen, Öffnungen zur Abgabe eines Mediums, zum Beispiel von Wasser und / oder Luft, angeordnet. Die Öffnungen zur Abgabe eines Mediums sind über zumindest eine Medienleitung in dem Handstück 17 mit der Steuer-, Regel- oder Versorgungseinheit 4 zum Zuführen zumindest eines Mediums verbunden.

In dem Instrumententeil 1 oder Handstück 17 ist des Weiteren eine mit elektrischer Energie betreibbare Speichervorrichtung 5 zur Speicherung von Identifikationsdaten und / oder Betriebsdaten und / oder Pflegedaten des Instrumententeils 1 oder Handstücks 17 angeordnet. Die Speichervorrichtung 5 kann im Handstückkopf 17A oder im Griffteil 17B angeordnet sein oder es kann jeweils ein Teil der Speichervorrichtung 5 im Handstückkopf 17A und im Griffteil 17B vorgesehen sein. Innerhalb des Griffteils 17B ist die Speichervorrichtung 5 oder ein Teil davon zum Beispiel an dessen proximalen oder freien Ende, insbesondere in oder an der Kupplungsvorrichtung 3, in einem unmittelbar an den Handstückkopf 17A anschließenden Abschnitt des Griffteils 17B oder in einer Biegung 18 des Griffteils 17B angeordnet.

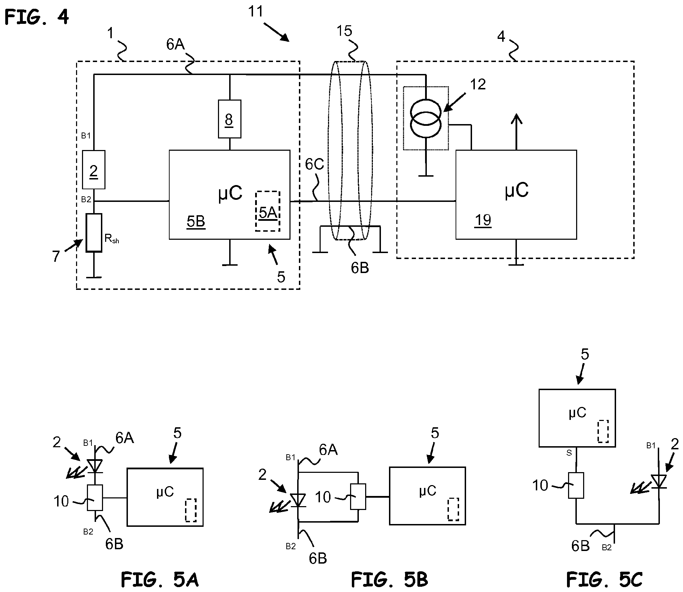

Die Speichervorrichtung 5 umfasst insbesondere ein Speicherelement 5A und einen Mikrocontroller 5B, insbesondere zum Betreiben des Speicherelements 5A (siehe Figuren 3 und 4).

Wie insbesondere aus den Figuren 3 und 4 zu erkennen ist, umfasst das Instrumententeil 1 oder das Handstück 17 des Weiteren zumindest eine (gemeinsame) elektrische Leitung 6A, 6B, die für die Versorgung der an dem Handstück 17 vorgesehenen Beleuchtungsvorrichtung 2 mit elektrischer Energie zum Betrieb der Beleuchtungsvorrichtung 2 und für die Versorgung der Speichervorrichtung 5 mit elektrischer Energie zum Betrieb der Speichervorrichtung 5 vorgesehen ist. Die zumindest eine (gemeinsame) elektrische Leitung 6A, 6B verbindet die Beleuchtungsvorrichtung 2 und die Speichervorrichtung 5 mit einer in der Steuer-, Regel- oder Versorgungseinheit 4 angeordneten elektrischen Energiequelle 12, insbesondere eine Konstantstromquelle, die ausgebildet ist, die Beleuchtungsvorrichtung 2 und die Speichervorrichtung 5 mit elektrischer Energie zu versorgen. Wie aus den Figuren 3 und 4 auch zu erkennen ist, erstreckt sich die zumindest eine (gemeinsame) elektrische Leitung 6A, 6B durch den Versorgungsschlauch 15 bis zu oder in die Steuer-, Regel- oder Versorgungseinheit 4.

Die zumindest eine (gemeinsame) elektrische Leitung 6A, 6B verbindet somit elektrisch die Beleuchtungsvorrichtung 2 und die Speichervorrichtung 5 mit der Steuer-, Regel- oder Versorgungseinheit 4, insbesondere deren elektrischer Energiequelle 12 und Mikrocontroller 19, wodurch ein elektrischer Schalt-, Steuer-, Regel- oder Versorgungskreis gebildet ist. Vorzugsweise umfasst der Schalt-, Steuer-, Regel- oder Versorgungskreis auch noch zumindest einen Sensor 10, wie im Folgenden noch im Detail beschrieben ist.

Die elektrische Energiequelle 12 in Form einer Konstantstromquelle versorgt das Instrumententeil 1 oder das Handstück 17 mit elektrischer Energie mit konstanter elektrischer Stromstärke. Dies bildet für die zumindest einen optischen Halbleiter umfassende Beleuchtungsvorrichtung 2 eine optimale Energieversorgung. Die Speichervorrichtung 5, insbesondere der Mikrocontroller 5B, benötigt jedoch eine elektrische Versorgung mit einer konstanten elektrischen Spannung. Dazu ist eine der Speichervorrichtung 5, insbesondere dem Mikrocontroller 5B, zugeordnete Vorrichtung 8 zur Spannungsaufbereitung vorgesehen, die ausgebildet ist, die von der elektrischen Energiequelle 12 (über die elektrische Leitung 6A, 6B) empfangene elektrischer Energie mit konstanter elektrischer Stromstärke in elektrische Energie mit konstanter elektrischer Spannung zu wandeln und der Speichervorrichtung 5 zuzuführen. Die Vorrichtung 8 zur Spannungsaufbereitung ist insbesondere in dem Instrumententeil 1 oder Handstück 17 angeordnet.

Gemäß der Figur 3 sind zwei (gemeinsame) elektrische Leitungen 6A, 6B für die Versorgung der Beleuchtungsvorrichtung 2 und der Speichervorrichtung 5 mit elektrischer Energie vorgesehen, so wie dies im Vorstehenden beschrieben ist. Zusätzlich sind die zwei elektrischen Leitungen 6A, 6B zum Übertragen von Identifikationsdaten und / oder Betriebsdaten und / oder Pflegedaten zwischen der Speichervorrichtung 5 und der mit dem Instrumententeil 1 oder Handstück 17 verbindbaren Steuer-, Regel- oder Versorgungseinheit 4 vorgesehen. In anderen Worten sind somit für die Versorgung der Beleuchtungsvorrichtung 2 und der Speichervorrichtung 5 mit elektrischer Energie und für die Datenübertragung nicht mehr als die zwei (gemeinsamen) elektrischen Leitungen 6A, 6B vorgesehen. Entsprechend ist die Steuer-, Regel- oder Versorgungseinheit 4 zum Austausch von Identifikationsdaten und / oder Betriebsdaten und / oder Pflegedaten des Instrumententeils 1 mit der Speichervorrichtung 5 ausgebildet.

Zur Übertragung von Identifikationsdaten und / oder Betriebsdaten und / oder Pflegedaten von der Speichervorrichtung 5 und zu der Steuer-, Regel- oder Versorgungseinheit 4, d.h. zum Auslesen von Daten aus der Speichervorrichtung 5, ist ein der Speichervorrichtung 5 zugeordnetes elektrisches Schaltelement 13 vorgesehen. Wie im Vorstehenden bereits beschrieben ist das Schaltelement 13 ausgebildet, Änderungen eines elektrischen Stromparameters zu bewirken, insbesondere der elektrischen Spannung oder der elektrischen Last, insbesondere durch das Kurzschließen eines Shunt-Widerstands 7. Die Änderungen des elektrischen Stromparameters definieren (digital) die übertragenen Daten. Die Steuer-, Regel- oder Versorgungseinheit 4, insbesondere deren Mikrocontroller 19, ist ausgebildet, die Änderungen des elektrischen Stromparameters zu detektieren oder auszugleichen und daraus die (digital) übertragenen Identifikationsdaten und / oder Betriebsdaten und / oder Pflegedaten zu erkennen oder auszulesen.

Im Instrumententeil 1 oder Handstück 17 ist des Weiteren einen Shunt-Widerstand 7 vorgesehen, der ausgebildet ist, Änderungen der elektrischen Stromstärke zur Übertragung von Identifikationsdaten und / oder Betriebsdaten und / oder Pflegedaten zwischen der Speichervorrichtung 5 und der mit dem Instrumententeil 1 verbindbaren Steuer-, Regel- oder Versorgungseinheit 4 aufzubereiten. Wie im Vorstehenden bereits beschrieben ist der Shunt-Widerstand 7 zum Abspeichern von Daten in der Speichervorrichtung 5 vorgesehen, insbesondere zur Übertragen von Daten von der Steuer-, Regel- oder Versorgungseinheit 4 zu der Speichervorrichtung 5.

Ein weiterer Shunt-Widerstand 20 ist der Steuer-, Regel- oder Versorgungseinheit 4, insbesondere dem Mikrocontroller 19 der Steuer-, Regel- oder Versorgungseinheit 4, zugeordnet.

Gemäß der Figur 4 sind zwei (gemeinsame) elektrische Leitungen 6A, 6B für die Versorgung der Beleuchtungsvorrichtung 2 und der Speichervorrichtung 5 mit elektrischer Energie vorgesehen, so wie dies im Vorstehenden beschrieben ist. Zum Übertragen von Identifikationsdaten und / oder Betriebsdaten und / oder Pflegedaten zwischen der Speichervorrichtung 5 und der mit dem Instrumententeil 1 oder Handstück 17 verbindbaren Steuer-, Regel- oder Versorgungseinheit 4 ist eine separate Leitung 6C oder Datenleitung vorgesehen. In anderen Worten sind somit für die Versorgung der Beleuchtungsvorrichtung 2 und der Speichervorrichtung 5 mit elektrischer Energie und für die Datenübertragung drei Leitungen vorgesehen, nämlich die zwei (gemeinsamen) elektrischen Leitungen 6A, 6B für die Versorgung mit elektrischer Energie und die separate Leitung 6C für die Übertragung der Daten, so wie dies im Vorstehenden beschrieben ist.

Die separate Datenleitung 6C kann als elektrische Leitung zum Übertragen elektrischer (Daten-)Signale oder als optische Leitung, zum Beispiel als Glasfaser, zum Übertragen optischer (Daten-)Signale ausgebildet sein. Die separate Leitung 6C erstreckt sich durch das Instrumententeil 1 oder Handstück 17 und den Versorgungsschlauch 15 bis zu oder in die Steuer-, Regel- oder Versorgungseinheit 4.

An der Kupplungsvorrichtung 3 des Instrumententeils 1 oder Handstücks 17, insbesondere an der Stirnfläche der Kupplungsvorrichtung 3, sind elektrische Kontakte 3A, 3B vorgesehen, die mit der zumindest einen gemeinsamen elektrischen Leitung 6A, 6B verbunden sind (siehe Figur 2). Diese lösbaren elektrischen Kontakte 3A, 3B verbinden die Beleuchtungsvorrichtung 2 und die Speichervorrichtung 5 elektrisch mit der Steuer-, Regel- oder Versorgungseinheit 4, insbesondere mit deren Energiequelle 12 und Mikrocontroller 19. Wenn nur zwei gemeinsame elektrische Leitungen 6A, 6B zur Übertragung der elektrischen Energie und von Daten vorgesehen sind, so wie dies im Vorstehenden beschrieben ist, dann sind in entsprechender Weise auch nur diese zwei elektrische Kontakte 3A, 3B an der Kupplungsvorrichtung 3 vorgesehen. Wenn zumindest eine separate optische oder elektrische Leitung 6C zum Übertragen von Identifikationsdaten und / oder Betriebsdaten und / oder Pflegedaten vorhanden ist, dann ist in entsprechender Weise ein weiterer optischer oder elektrischer Kontakt 3C vorgesehen, der mit dieser zumindest einen separaten Leitung 6C für die Datenübertragung verbunden ist.

Vorzugsweise ist der zumindest eine elektrische Kontakt oder sind die elektrischen Kontakte lösbar mit der Steuer-, Regel- oder Versorgungseinheit 4 verbunden. Der zumindest eine elektrische Kontakt ist zum Beispiel als stiftförmiger elektrischer Kontakt, als Federkontakt, als Schleifkontakt oder als ringförmiger, ein Bauteil der Kupplungsvorrichtung umgebender elektrischer Kontakt ausgebildet.

In dem medizinischen oder dentalen Instrumententeil 1 oder Handstück 17 ist vorzugsweise zumindest einen Sensor 10 vorgesehen, der ausgebildet ist, einen Betriebszustand des Instrumententeils 1 oder Handstücks 17 zu detektieren und ein Sensorsignal zu erzeugen. Der Sensor 10 umfasst zum Beispiel einen Temperatursensor oder einen Drehzahlsensor zum Messen der Drehzahl eines Antriebselements der Instrumententeils 1 / Handstücks 17. In den Figuren 5A - 5C sind unterschiedliche Anordnungen des Sensors 10 dargestellt.

Der zumindest eine Sensor 10 ist mit der zumindest einen elektrischen Leitung 6A, 6B elektrisch verbunden, so dass das Sensorsignal des Sensors 10 und / oder elektrische Energie zum Betreiben des Sensors 10 über die zumindest eine elektrische Leitung 6A, 6B übertragbar ist/sind.

Der zumindest eine Sensor 10 ist vorzugsweise auch mit der Speichervorrichtung 5, insbesondere dem Mikrocontroller 5B, elektrisch verbunden, so dass insbesondere ein Sensorsignal des Sensors 10 an die Speichervorrichtung 5 weiterleitbar ist. Die Speichervorrichtung 5, insbesondere der Mikrocontroller 5B, sind wahlweise ausgebildet (1) das (analoge) Sensorsignal des Sensors 10 weiterzuleiten, vorzugsweise an die Steuer-, Regel- oder Versorgungseinheit 4, insbesondere an deren Mikrocontroller 19, oder (2) das analoge Sensorsignal in ein digitales Signal zu wandeln und an die Steuer-, Regel- oder Versorgungseinheit 4, insbesondere an deren Mikrocontroller 19, zu leiten oder (3) das (analoge) Sensorsignal des Sensors 10 zu empfangen und zu verarbeiten und das Instrumententeil 1 / Handstück 17 oder ein Bauteil davon auf Basis des Sensorsignals zu betreiben, zu regeln oder zu steuern. Das Weiterleiten des Sensorsignals an die Steuer-, Regel- oder Versorgungseinheit 4 gemäß (1) oder (2) erfolgt vorzugsweise über zumindest eine der Leitungen 6A, 6B, 6C.

Bei der Ausführungsform der Figur 5A ist der Sensor 10, vorzugsweise ein Sensor zum Messen der Drehzahl, elektrisch in Serie mit der Beleuchtungsvorrichtung 2 angeordnet. Bei der Ausführungsform der Figur 5B ist der Sensor 10, vorzugsweise ein Sensor zum Messen der Temperatur, elektrisch parallel mit der Beleuchtungsvorrichtung 2 angeordnet. In beiden Ausführungsformen weisen der Sensor 10 und die Beleuchtungsvorrichtung 2 eine gemeinsame elektrische Energiequelle 12 auf und sie sind mit den elektrischen Leitung 6A, 6B elektrisch verbunden.

Bei der Ausführungsform der Figur 5C sind der Sensor 10, vorzugsweise ein Sensor zum Messen der Temperatur, und die Beleuchtungsvorrichtung 2 mit unterschiedlichen elektrischen Energiequellen zur Versorgung mit elektrischer Energie verbunden.

Die Erfindung ist nicht auf die dargestellten und beschriebenen Ausführungsbeispiele beschränkt. Des Weiteren sind alle Merkmale aller beschriebenen und dargestellten Ausführungsbeispiele miteinander kombinierbar.

## Patentansprüche

1. Medizinisches oder dentales Instrumententeil (1), umfassend: eine Kupplungsvorrichtung (3) zur Verbindung des Instrumententeils (1) mit einer Steuer-, Regel- oder Versorgungseinheit (4), und eine mit elektrischer Energie betreibbare Speichervorrichtung (5) zur Speicherung von Identifikationsdaten und / oder Betriebsdaten und / oder Pflegedaten des Instrumententeils (1), **gekennzeichnet durch**
exakt drei elektrische Leitungen (6A, 6B, 6C), wobei von diesen exakt drei elektrischen Leitungen (6A, 6B, 6C) eine erste und zweite elektrische Leitung (6A, 6B) für die Versorgung einer an dem Instrumententeil (1) vorgesehenen oder mit dem Instrumententeil (1) verbindbaren Beleuchtungsvorrichtung (2) mit elektrischer Energie zum Betrieb der Beleuchtungsvorrichtung (2) und für die Versorgung der Speichervorrichtung (5) mit elektrischer Energie zum Betrieb der Speichervorrichtung (5) vorgesehen sind, und wobei eine dritte elektrische Leitung (6C) der exakt drei elektrischen Leitungen (6A, 6B, 6C) zum Übertragen von Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) vorgesehen ist.

2. Medizinisches oder dentales Instrumententeil (1) nach Anspruch 1, **gekennzeichnet durch**
eine der Speichervorrichtung (5) zugeordnete Vorrichtung (8) zur Spannungsaufbereitung, die ausgebildet ist, der Speichervorrichtung (5) eine konstante elektrische Spannung zuzuführen.

3. Medizinisches oder dentales Instrumententeil (1) nach Anspruch 2, **dadurch gekennzeichnet, dass**
die erste und zweite elektrische Leitung (6A, 6B) die Beleuchtungsvorrichtung (2) und die Speichervorrichtung (5) mit einer als Konstantstromquelle ausgebildeten Energiequelle (12) verbindet, die Energie mit einer konstanten Stromstärke zu Verfügung stellt, wobei die Vorrichtung (8) zur Spannungsaufbereitung ausgebildet ist, die von der Konstantstromquelle (12) empfangene Energie mit einer konstanten Stromstärke in elektrische Energie mit konstanter elektrischer Spannung zu wandeln und der Speichervorrichtung (5) zuzuführen.

4. Medizinisches oder dentales Instrumententeil (1) nach einem der vorstehenden Ansprüche, **gekennzeichnet durch**
eine der Beleuchtungsvorrichtung (2) zugeordnete Vorrichtung zur Spannungsüberwachung, die ausgebildet ist, die der Beleuchtungsvorrichtung (2) zugeführte elektrische Spannung zu überwachen.

5. Medizinisches oder dentales Instrumententeil (1) nach einem der vorstehenden Ansprüche, **gekennzeichnet durch**
zumindest einen Sensor (10), der ausgebildet ist, einen Betriebszustand des Instrumententeils (1) zu detektieren und ein Sensorsignal zu erzeugen, wobei der zumindest eine Sensor (10) mit der ersten und zweiten elektrischen Leitung (6A, 6B) elektrisch verbunden ist, so dass elektrische Energie zum Betreiben des Sensors (10) über die erste und zweite elektrische Leitung (6A, 6B) übertragbar ist.

6. Medizinisches oder dentales Instrumententeil (1) nach Anspruch 5, **dadurch gekennzeichnet, dass**
der zumindest eine Sensor (10) mit der dritten elektrischen Leitung (6C) verbunden ist, so dass Sensordaten vom Sensor (10) über die dritte elektrische Leitung (6C) an die Steuer-, Regel- oder Versorgungseinheit (4) übertragbar sind.

7. Medizinisches oder dentales Instrumententeil (1) nach Anspruch 5 oder 6, **dadurch gekennzeichnet, dass**
der zumindest eine Sensor (10) einen Temperatursensor zum Messen der Temperatur eines Abschnitts des Instrumententeils (1) oder eines in dem Instrumententeil (1) angeordneten Bauteils umfasst.

8. Medizinisches oder dentales Instrumententeil (1) nach Anspruch 7, **dadurch gekennzeichnet, dass**
der zumindest eine Sensor (10) ein Material, dessen elektrischer Widerstand sich mit der Temperatur verändert, insbesondere einen NTC-Temperatursensor, umfasst.

9. Medizinisches oder dentales Instrumententeil (1) nach Anspruch 5 oder 6, **dadurch gekennzeichnet, dass**
der zumindest eine Sensor (10) einen Drehzahlsensor zum Messen der Drehzahl eines Antriebselements des Instrumententeils (1) oder eines damit verbindbaren Werkzeugs aufweist.

10. Medizinisches oder dentales Instrumententeil (1) nach einem der Ansprüche 5 - 9, **dadurch gekennzeichnet, dass**
der zumindest eine Sensor (10) mit der Speichervorrichtung (5) elektrisch verbunden ist, so dass ein Sensorsignal des zumindest einen Sensors (10) an die Speichervorrichtung (5) weiterleitbar ist.

11. Medizinisches oder dentales Instrumententeil (1) nach einem der vorstehenden Ansprüche, **dadurch gekennzeichnet, dass**
die Speichervorrichtung (5) zum aktiven Ausgeben eines digitalen Signals oder von digitalen Daten ausgebildet ist.

12. Medizinische oder dentale Behandlungsvorrichtung (11), **gekennzeichnet durch** ein medizinisches oder dentales Instrumententeil (1) nach einem der vorstehenden Ansprüche, eine elektrische Energiequelle (12), die ausgebildet ist, die Beleuchtungsvorrichtung (2) und die Speichervorrichtung (5) über die erste und zweite elektrische Leitung (6A, 6B) der exakt drei elektrischen Leitungen (6A, 6B, 6C) mit elektrischer Energie zu versorgen, und eine Steuer-, Regel- oder Versorgungseinheit (4), die zum Austausch von Identifikationsdaten und / oder Betriebsdaten und / oder Pflegedaten des Instrumententeils (1) mit der Speichervorrichtung (5) des Instrumententeils (1) über die dritte elektrische Leitung (6C) der exakt drei elektrischen Leitungen (6A, 6B, 6C) ausgebildet ist.

13. Medizinische oder dentale Behandlungsvorrichtung (11) nach Anspruch 12, **dadurch gekennzeichnet, dass**
die elektrische Energiequelle (12) durch eine Konstantstromquelle gebildet ist, welche für die Beleuchtungsvorrichtung (2) und die Speichervorrichtung (5) Energie mit einer konstanten Stromstärke zu Verfügung stellt.

14. Medizinische oder dentale Behandlungsvorrichtung (11) nach Anspruch 12 oder 13 **dadurch gekennzeichnet, dass**
die Steuer-, Regel- oder Versorgungseinheit (4) ausgebildet ist, das Instrumententeil (1) auf Basis von in der Speichervorrichtung (5) gespeicherten und aus der Speichervorrichtung (5) ausgelesenen Identifikationsdaten und / oder Betriebsdaten und / oder Pflegedaten und / oder auf Basis des von dem zumindest einem Sensor (10) erhaltenen Sensorsignals zu betreiben.

15. Verfahren zum Betrieb eines medizinischen oder dentalen Instrumententeils (1), nach einem der Ansprüche 1 - 11 oder einer medizinischen oder dentalen Behandlungsvorrichtung (11) nach einem der Ansprüche 12 - 14, **dadurch gekennzeichnet, dass**
die erste und zweite elektrische Leitung (6A, 6B) der exakt drei elektrischen Leitungen (6A, 6B, 6C) eine an dem Instrumententeil (1) vorgesehene oder mit dem Instrumententeil (1) verbindbare Beleuchtungsvorrichtung (2) zum Betrieb der Beleuchtungsvorrichtung (2) und eine Speichervorrichtung (5) zum Betrieb der Speichervorrichtung (5) mit elektrischer Energie versorgt und die dritte elektrische Leitung (6C) der exakt drei elektrischen Leitungen (6A, 6B, 6C) Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) überträgt.

## Claims

1. A medical or dental instrument part (1), comprising: a coupling device (3) for connecting the instrument part (1) to a control, regulating or supply unit (4), and a memory device (5) that can be operated with electrical power for storing identification data and/or operating and/or care data of the instrument part (1), **characterized by** exactly three electrical lines (6A, 6B, 6C), wherein a first and second electrical line (6A, 6B) of these exactly three electrical lines (6A, 6B, 6C) are provided for supplying electrical power to a lighting device (2) that is provided on the instrument part (1) or can be connected to the instrument part (1) for operating the lighting device (2) and for supplying electrical power to the memory device (5) for operating the memory device (5), and wherein a third electrical line (6C) of the exactly three electrical lines (6A, 6B, 6C) is provided for transmitting identification data and/or operating data and/or care data between the memory device (5) and the control, regulating or supply unit (4) connectable to the instrument part (1).

2. The medical or dental instrument part (1) according to claim 1, **characterized by** a device for voltage processing (8) assigned to the memory device (5) and configured to supply a constant electrical voltage to the memory device (5).

3. The medical or dental instrument part (1) according to claim 2, **characterized in that** the first and second electrical lines (6A, 6B) connect the lighting device (2) and the memory device (5) to an energy source (12) configured as a constant current source, which provides electrical power at a constant electrical amperage, wherein the device for voltage processing (8) is configured to convert the electrical power having a constant electrical amperage and received from the constant current source (12) into electrical power with a constant electrical voltage and to supply it to the memory device (5).

4. The medical or dental instrument part (1) according to any one of the preceding claims, **characterized by**
a device for monitoring voltage which is assigned to the lighting device (2) and is configured to monitor the electrical voltage supplied to the lighting device (2).

5. The medical or dental instrument part (1) according to any one of the preceding claims, **characterized by**
at least one sensor (10), which is configured to detect an operating state of the instrument part (1) and to generate a sensor signal, wherein the at least one sensor (10) is electrically connected to the first and second electrical line (6A, 6B) so that electrical power for operating the sensor (10) can be transmitted over the first and second electrical line (6A, 6B).

6. The medical or dental instrument part (1) according to claim 5, **characterized in that** the at least one sensor (10) is connected to the third electrical line (6C), so that sensor data can be transmitted from the sensor (10) over the third electrical line (6C) to the control, regulating or supply unit (4).

7. The medical or dental instrument part (1) according to claim 5 or 6, **characterized in that**
the at least one sensor (10) comprises a temperature sensor for measuring the temperature of a section of the instrument part (1) or of a component disposed in the instrument part (1).

8. The medical or dental instrument part (1) according to claim 7, **characterized in that** the at least one sensor (10) comprises a material whose electrical resistance changes with the temperature, in particular an NCT temperature sensor.

9. The medical or dental instrument part (1) according to claim 5 or 6, **characterized in that**
the at least one sensor (10) comprises a speed sensor for measuring the rotational speed of a drive element of the instrument part (1) or a tool that can be connected thereto.

10. The medical or dental instrument part (1) according to one of the claims 5 - 9, **characterized in that**
the at least one sensor (10) is electrically connected to the memory device (5), so that a sensor signal of the at least one sensor (10) can be transmitted to the memory device (5).

11. The medical or dental instrument part (1) according to any one of the preceding claims, **characterized in that**
the memory device (5) is configured to actively output a digital signal or digital data.

12. A medical or dental treatment device (11), **characterized by**
a medical or dental instrument part (1) according to any one of the preceding claims, an electrical power source (12) which is configured to supply electrical power to the lighting device (2) and the memory device (5) via the first and second electrical line (6A, 6B) of the exactly three electrical lines (6A, 6B, 6C), and a control, regulating or supply unit (4), which is configured to exchange identification data and/or operating data and/or care data of the instrument part (1) with the memory device (5) of the instrument part (1) via the third electrical line (6C) of the exactly three electrical lines (6A, 6B, 6C).

13. The medical or dental treatment device (11) according to claim 12, **characterized in that**
the electrical energy source (12) is formed by a constant current source which provides energy at a constant amperage for the lighting device (2) and the memory device (5).

14. The medical or dental treatment device (11) according to claim 12 or 13, **characterized in that**
the control, regulating or supply unit (4) is configured to operate the instrument part (1) on the basis of identification data and/or operating data and/or care data stored in the memory device (5) and read out of the memory device (5) and/or on the basis of the sensor signal received from the at least one sensor (10).

15. A method for operating a medical or dental instrument part (1) according to any one of claims 1 - 11 or a medical or dental treatment device (11) according to any one of claims 12 - 14, **characterized in that**
the first and second electric line (6A, 6B) of the exactly three electric lines (6A, 6B, 6C) supply electric power to a lighting device (2) provided on the instrument part (1) or connectable to the instrument part (1) for operating the lighting device (2) and to a memory device (5) for operating the memory device (5), and the third electric line (6C) of the exactly three electric lines (6A, 6B, 6C) transmits identification data and/or operation data and/or care data between the memory device (5) and the control, regulation or supply unit (4) connectable to the instrument part (1).

## Revendications

1. Partie d'instrument médical ou dentaire (1) comprenant: un dispositif d'accouplement (3) pour relier la partie d'instrument (1) à une unité de commande, de régulation ou d'alimentation (4) et un dispositif de mise en mémoire (5) pouvant fonctionner avec de l'énergie électrique pour la mise en mémoire de données d'identification et/ou de données de fonctionnement et/ou de données de soins de la partie d'instrument (1), **caractérisée par**
exactement trois lignes électriques (6A, 6B, 6C), dans laquelle, de ces exactement trois lignes électriques (6A, 6B, 6C), une première et une deuxième ligne électrique (6A, 6B) sont prévues pour l'alimentation d'un dispositif d'éclairage (2) prévu sur la partie d'instrument (1), ou pouvant être relié à la partie d'instrument (1), avec de l'énergie électrique pour le fonctionnement du dispositif d'éclairage (2) et pour l'alimentation du dispositif de mise en mémoire (5) avec de l'énergie électrique pour le fonctionnement du dispositif de mise en mémoire (5), et dans laquelle une troisième ligne électrique (6C) des exactement trois lignes électriques (6A, 6B, 6C) est prévue pour la transmission de données d'identification et/ou de données de fonctionnement et/ou de données de soins entre le dispositif de mise en mémoire (5) et l'unité de commande, de régulation ou d'alimentation (4) pouvant être reliée à la partie d'instrument (1).

2. Partie d'instrument médical ou dentaire **(1)** selon la revendication 1, **caractérisée par** un dispositif pour la préparation de tension (8), associé au dispositif de mise en mémoire (5), lequel est réalisé pour amener une tension électrique constante au dispositif de mémoire (5).

3. Partie d'instrument médical ou dentaire (1) selon la revendication 2, **caractérisée en ce que**
les première et deuxième lignes électriques (6A, 6B) relient le dispositif d'éclairage (2) et le dispositif de mise en mémoire (5) à une source d'énergie (12), réalisée en tant que source de courant constant, laquelle met à disposition de l'énergie avec une intensité de courant constante, dans laquelle le dispositif (8) pour la préparation de tension est réalisé pour convertir l'énergie d'une intensité de courant constante réceptionnée par la source de courant constant (12) en énergie électrique d'une tension électrique constante et l'amener au dispositif de mise en mémoire (5).

4. Partie d'instrument médical ou dentaire (1) selon l'une des revendications précédentes, **caractérisée par**
un dispositif pour la surveillance de tension associé au dispositif d'éclairage (2), lequel est réalisé pour surveiller la tension électrique amenée au dispositif d'éclairage (2).

5. Partie d'instrument médical ou dentaire (1) selon l'une des revendications précédentes, **caractérisée par**
au moins un capteur (10) qui est réalisé pour détecter un état de fonctionnement de la partie d'instrument (1) et générer un signal de capteur, dans laquelle l'au moins un capteur (10) est en liaison électrique avec les première et deuxième lignes électriques (6A, 6B) de sorte que de l'énergie électrique pour le fonctionnement du capteur (10) peut être transmise via les première et deuxième lignes électriques (6A, 6B).

6. Partie d'instrument médical ou dentaire (1) selon la revendication 5, **caractérisée en ce que**
l'au moins un capteur (10) est relié à la troisième ligne électrique (6C) de sorte que des données de capteur peuvent être transmises par le capteur (10) via la troisième ligne électrique (6C) à l'unité de commande, de régulation ou d'alimentation (4).

7. Partie d'instrument médical ou dentaire (1) selon la revendication 5 ou 6, **caractérisée en ce que**
l'au moins un capteur (10) comprend un capteur de température pour mesurer la température d'une portion de la partie d'instrument (1) ou d'une pièce disposée dans la partie d'instrument (1).

8. Partie d'instrument médical ou dentaire (1) selon la revendication 7, **caractérisée en ce que**
l'au moins un capteur (10) comprend un matériau dont la résistance électrique varie avec la température, en particulier un capteur de température NTC.

9. Partie d'instrument médical ou dentaire (1) selon la revendication 5 ou 6, **caractérisée en ce que**
l'au moins un capteur (10) présente un capteur de vitesse de rotation pour mesurer la vitesse de rotation d'un élément d'entraînement de la partie d'instrument (1) ou d'un outil pouvant y être relié.

10. Partie d'instrument médical ou dentaire (1) selon l'une des revendications 5 - 9, **caractérisée en ce que**
l'au moins un capteur (10) est en liaison électrique avec le dispositif de mise en mémoire (5) de sorte qu'un signal de capteur de l'au moins un capteur (10) peut être retransmis au dispositif de mise en mémoire (5).

11. Partie d'instrument médical ou dentaire (1) selon l'une des revendications précédentes, **caractérisée en ce que**
le dispositif de mise en mémoire (5) est réalisé pour l'émission active d'un signal numérique ou de données numériques.

12. Dispositif de traitement médical ou dentaire (11), **caractérisé par** une partie d'instrument médical ou dentaire (1) selon l'une des revendications précédentes, une source d'énergie électrique (12) réalisée pour alimenter en énergie électrique le dispositif d'éclairage (2) et le dispositif de mise en mémoire (5) via les première et deuxième lignes électriques (6A, 6B) des exactement trois lignes électriques (6A, 6B, 6C), et une unité de commande, de régulation ou d'alimentation (4) réalisée pour l'échange de données d'identification et/ou de données de fonctionnement et/ou de données de soins de la partie d'instrument (1) avec le dispositif de mise en mémoire (5) de la partie d'instrument (1) via la troisième ligne électrique (6C) des exactement trois lignes électriques (6A, 6B, 6C).

13. Dispositif de traitement médical ou dentaire (11) selon la revendication 12, **caractérisé en ce que**
la source d'énergie électrique (12) est formée par une source de courant constant, laquelle met à disposition du dispositif d'éclairage (2) et du dispositif de mise en mémoire (5) de l'énergie avec une intensité de courant constante.

14. Dispositif de traitement médical ou dentaire (11) selon la revendication 12 ou 13, **caractérisé en ce que**
l'unité de commande, de régulation ou d'alimentation (4) est réalisée pour faire fonctionner la partie d'instrument (1) sur la base de données d'identification et/ou de données de fonctionnement et/ou de données de soins mises en mémoire dans le dispositif de mise en mémoire (5) et lues à partir du dispositif de mise en mémoire (5) et/ou sur la base du signal de capteur obtenu de l'au moins un capteur (10).

15. Procédé de fonctionnement d'une partie d'instrument médical ou dentaire (1) selon l'une des revendications 1 - 11 ou d'un dispositif de traitement médical ou dentaire (11) selon l'une des revendications 12 - 14, **caractérisé en ce que** les première et deuxième lignes électriques (6A, 6B) des exactement trois lignes électriques (6A, 6B, 6C) alimentent en énergie électrique un dispositif d'éclairage (2) prévu sur la partie d'instrument (1), ou pouvant être relié à la partie d'instrument (1), pour faire fonctionner le dispositif d'éclairage (2) et un dispositif de mise en mémoire (5) pour faire fonctionner le dispositif de mise en mémoire (5), et la troisième ligne électrique (6C) des exactement trois lignes électriques (6A, 6B, 6C) transmet des données d'identification et/ou des données de fonctionnement et/ou des données de soins entre le dispositif de mise en mémoire (5) et l'unité de commande, de régulation ou d'alimentation (4) pouvant être reliée à la partie d'instrument (1).
